(19) Europäisches Patentamt
European Patent Office
Office européen des brevets

(11) Veröffentlichungsnummer: **0 018 609**
**B1**

(12) # EUROPÄISCHE PATENTSCHRIFT

(45) Veröffentlichungstag der Patentschrift: **21.09.83**

(21) Anmeldenummer: **80102252.6**

(22) Anmeldetag: **25.04.80**

(51) Int. Cl.³: **A 61 K 37/02,** A 61 K 37/26, C 07 C 103/52, C 07 G 7/00, C 09 K 15/14

(54) **Gegen Denaturierung beständige, wässrige Protein-Lösungen, Verfahren zu ihrer Herstellung und ihre Verwendung.**

(30) Priorität: **30.04.79 DE 2917535**
**22.12.79 DE 2952119**

(43) Veröffentlichungstag der Anmeldung: **12.11.80 Patentblatt 80/23**

(45) Bekanntmachung des Hinweises auf die Patenterteilung: **21.09.83 Patentblatt 83/38**

(84) Benannte Vertragsstaaten: **AT BE CH DE FR GB IT LI NL SE**

(56) Entgegenhaltungen:
**DE-A-2 212 695**
**DE-A-2 620 483**
**DE-A-2 641 819**
**US-A-4 179 337**

**Biochemical Journal, Band 117, 1970 Cambridge R. Cecil et al. «Protein-Hydrocarbon Interactions»**

**Biochimica et Biophysica Acta, Band 541, 1978 B. Schobert et al. «Unusual Solution Properties of Proline and its Interaction with Proteins» Seiten 270 bis 277**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Thurow, Horst, Dr., Parkstrasse 20, D-6233 Kelkheim (Taunus) (DE)**

**EP 0 018 609 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Gegen Denaturierung beständige, wässrige Protein-Lösungen, Verfahren zu ihrer Herstellung und Ihre Verwendung

Die Erfindung betrifft eine wässrige Proteinlösung, die gegen Denaturierung der Proteine an Grenzflächen beständig ist, sowie Verfahren zur Herstellung einer solchen Lösung und zur Behandlung von Oberflächen, die auf Proteinlösungen denaturierend wirken.

Es ist bekannt, dass gelöste Proteine an hydrophoben Grenzflächen (dazu gehört auch die Grenzfläche wässrige Lösung/Luft) adsorbiert werden (C.W.N. Cumper und A.E. Alexander, Trans Faraday Soc. 46, 235 (1950)). Proteine sind amphiphile Substanzen, d.h. sie haben sowohl hydrophile als auch hydrophobe Bereiche. Die hydrophoben Bereiche bilden den Kontakt zur hydrophoben Grenzfläche.

Als Folge der Adsorption der Proteine an Grenzflächen werden verschiedene Sekundärreaktionen beobachtet, die man allgemein unter dem Begriff «Denaturierung» zusammenfasst. Es kommt zu einer Formänderung der adsorbierten Proteinmoleküle (Änderung der Tertiär- und/oder Sekundärstruktur). Daneben kann es zur Aggregation von adsorbierten Proteinmolekülen zu löslichen oder unlöslichen polymeren Formen kommen. So ist von vielen Proteinen eine Oberflächen-Aggregation bekannt, die sich z.B. als Trübung der Lösung oder als biologische Inaktivierung der Proteine beim Rühren oder Schütteln der wässrigen Lösungen bemerkbar macht (A.F. Henson, I.R. Mitchell, P.R. Mussellwhite, J. Colloid Interface Sci. 32, 162 (1970)). Diese Oberflächenadsorption und -aggregation ist besonders nachteilig in Apparaten zum Transport von Proteinlösungen z.B. in automatischen Dosiergeräten für Arzneimittel. In einigen Fällen kommt es auch zu chemischen Reaktionen der adsorbierten Proteine mit gelösten Substanzen (F. MacRitchie, J. Macromol. Sci., Chem., 4, 1169 (1970)).

Das gilt insbesondere für Rinder-, Schweine-, Humaninsulin oder deren Des-B1-Phenylalaninderivate. Diese sind mit einem Zinkgehalt bis 0,8%, bezogen auf das Insulingewicht, in wässrigen Medien unterhalb von pH 4,3 und oberhalb von pH 6,5 klar gelöst. Die genannten Insuline bilden in wässriger Lösung Aggregate, so dass die Lösung einen Gleichgewichtszustand aus monomeren, dimeren, tetrameren, hexameren und oligomeren Insulinmolekülen darstellt. Es ist bekannt, dass Insulin an hydrophoben Oberflächen, wozu auch die Grenzfläche Lösung/Luft zählt, stark absorbiert wird (Weisenfeld et al., Diabets 17, 766 (1968) und Browne et al., Eur.J.Biochem. 33 233 (1973)). Eigene Versuche haben ergeben, dass das absorbierte Insulin an der Oberfläche denaturieren kann. Dieser Prozess wird durch Temperatur und Bewegung der Lösung beeinflusst. Das denaturierte Produkt wird als polymeres Aggregat wieder desorbiert und fällt bei einer genügend hohen Konzentration in der Lösung als Niederschlag aus oder bildet ein thixotropes Gel. Das denaturierte Insulin ist biologisch unwirksam und führt zu Verstopfungen von Förderschläuchen, z.B. in einer kontinuierlich oder gesteuert arbeitenden Insulin-Infusionspumpe, wie sie in künstlichen Betazellen verwendet wird.

Daneben kann das denaturierte Insulin zu immunologischen Unverträglichkeiten Anlass geben. Es sind Untersuchungen bekannt, die die physikalische Zustandsform von Insulin für die Bildung von Antikörpern gegen Insulin verantwortlich machen (Kumar et al. Horm. Metab. Res. 6, 175 (1974)). Es ist darüber hinaus bekannt, dass selbst Humaninsulin, beim Menschen angewandt, zu immunologischen Reaktionen führen kann (A. Teuscher et al., Diabetologia 13, 435 (1977)).

Nach dem Stand der Technik hergestellte wässrige Insulinlösungen für therapeutische Zwecke können neben dem Wirkstoff, Rinder- oder Schweineinsulin oder einem Des-B1-Phenylalaninderivate dieser Insuline, gelöstes Zink bis zu 0,8%, bezogen auf das Insulingewicht, ein Mittel zu Einstellung der Isotonie, wie Natriumchlorid, Glycerin oder Glucose, ein Konservierungsmittel, wie Phenol, Kresol oder p-Hydroxybenzoesäuremethylester, und ein Salz zur Pufferung des pH-Wertes, wie Natriumphosphat, Acetat oder Citrat, enthalten. Daneben können noch Depothilfsstoffe, wie Protamin oder Surfen®, zur Erzielung einer verzögerten Insulinwirkung zugesetzt sein oder die Lösungen sind mit kristallinen oder amorphen Depotformen des Insulins gemischt. Es wurde festgestellt, dass gelöstes Insulin in allen diesen Präparaten an Grenzflächen denaturiert.

Eigene Versuche haben ergeben, dass die Denaturierungsgeschwindigkeit mit der Temperatur, Bewegung der Lösung und dem pH-Wert der Lösungen anstiegen. Humaninsulin denaturierte in wässriger Lösung ebenfalls. Zusätze, die die Aggregationsgleichgewichte des Insulins in wässriger Lösung in Richtung des monomeren Moleküls verschieben, wie Guanidin, Harnstoff, Pyridin oder monomere Detergenzien, beschleunigten die Denaturierung. Substanzen, die die Gleichgewichte in die entgegengesetzte Richtung verschieben, wie Zink und andere zweiwertige Metallionen, verzögerten die Denaturierung. Aber selbst eine Kombination aller günstigen Bedingungen konnte die Denaturierung von Insulin nicht verhindern. Diese Denaturierung war auch, wenn auch langsamer, bei ruhender Lagerung der Lösungen zu beobachten.

Eine spezielle Form der hydrophoben Grenzflächen bildet sich beim Gefrieren von wässrigen Lösungen, z.B. bei der Gefriertrocknung von Proteinen. An diesen Grenzflächen kommt es ebenfalls zu den beschriebenen Denaturierungen von Proteinen (z.B. Hansson, Acta Chem. Scand., 22, 483 (1968)).

Der Denaturierungsprozess kann einem Protein immunogene Eigenschaften (d.h. die Fähigkeit, immunologische Abwehrreaktionen in einem Organismus zu induzieren) verleihen oder bereits vorhandene immunogene Eigenschaften verstärken. Ausserdem können biologische Eigenschaften, wie enzymatische, serologische oder hormonelle Aktivitäten, verändert oder zerstört werden.

Es ist ferner bekannt, dass man die Adsorption von Proteinen an die Grenzfläche, die sich zwischen einer wässrigen Lösung und einer flüssigen hydrophoben Phase ausbildet, dadurch verhindern kann, dass man diesem System monomere oberflächenaktive Substanzen, wie Alkylalkohole, zusetzt (R. Cecil und C.F. Louis, Biochem. J. 117, 147 (1970)). Diese Substanzen werden ihrerseits reversibel an die hydrophoben Grenzflächen adsorbiert und verdrängen so die Proteine. Nachteilig bei diesem Verfahren ist, dass die Anwendungskonzentration der oberflächenaktiven Substanzen nahe deren Sättigungsgrenze in wässriger Lösung liegen muss, denn nur so wird eine optimale Beladung der Grenzfläche gewährleistet. In vielen Fällen ist die Grösse der Grenzfläche nicht konstant, sondern variabel (z.B. die Grenzfläche Lösung/Luft beim Rühren oder Schütteln von Proteinlösungen), so dass die wässrige Lösung Moleküle dieser oberflächenaktiven Substanzen abwechselnd nachliefern und aufnehmen muss, d.h. einen Puffervorrat enthalten muss. Bei einer notwendigen Anwendungskonzentration nahe der Sättigungsgrenze ist dies jedoch nur begrenzt möglich.

Ein weiterer Nachteil dieses Verfahrens ist ferner, dass die erwähnten monomeren oberflächenaktiven Substanzen nicht nur an den hydrophoben Grenzflächen adsorbiert werden, sondern auch an den hydrophoben Bereichen der gelösten Proteine. Dort bewirken sie eine Denaturierung der gelösten Proteine, die in vielen Fällen irreversibel ist und die man gerade verhindern will.

Die Stärke der Bindung der monomeren oberflächenaktiven Substanzen sowohl an die hydrophoben Grenzflächen als auch an die hydrophoben Bereiche der gelösten Proteine ist von der Hydrophobizität der Substanzen, d.h. z.B. von der Länge der Alkylketten, abhängig. Je länger die Alkylkette, um so grösser die Bindungsstärke. Der Widerspruch zwischen einer möglichst vollständigen Beladung der Grenzflächen mit den oberflächenaktiven Substanzen (erreichbar durch hohe Hydrophobizität der Substanzen oder hoher Anwendungskonzentration) und möglichst geringer Bindung an die hydrophoben Bereiche der gelösten Proteine schien nicht lösbar zu sein.

Weiterhin sind aus der DE-A-2 620 483 wässrige Insulinlösungen bekannt, bei denen durch Zusatz von 0,1 bis 20 Gew.-% Polyoxyethylenethern höhere Alkohole ein Schutz gegen Gelbildung und Ausfällung während der Lagerung bezweckt wird.

Bei Verwendung dieser Polyoxyethylenether, in deren Molekülkette sich Ethyleneinheiten mit Sauerstoffatomen abwechseln, konnte keine Schutzwirkung gegen die Denaturierung wässriger Proteinlösungen beobachtet werden. Die genannten Ethyleneinheiten haben offensichtlich nicht die für eine solche Schutzwirkung notwendigen hydrophoben Eigenschaften, da sie durch ihre räumliche Anordnung in der Kette nicht mit den hydrophoben Grenzflächen bzw. mit den hydrophoben Bereichen gelöster Proteine in Wechselwirkung treten können.

Es wurde nun gefunden, dass polymere Substanzen mit alternierend angeordneten hydrophoben und hydrophilen Bereichen die Grenzflächen derart verändern, dass die Adsorption von Proteinen an diese Grenzflächen und dadurch auch die erwähnten Sekundärreaktionen, wie z.B. die Grenzflächenaggregation, wirksam verhindert werden.

Gegenstand der Erfindung ist demgemäss eine wässrige Proteinlösung, gekennzeichnet durch einen Gehalt an einer oberflächenaktiven Substanz mit kettenförmiger Grundstruktur, deren Glieder schwach hydrophobe und schwach hydrophile Bereiche in alternierender Anordnung enthalten. Insbesondere sind Gegenstand der Erfindung wässrige Insulinlösungen ggf. mit üblichen Zusätzen zur Einstellung der Isotonie, Konservierung und/oder Depot-Wirkung, die durch den Zusatz einer solchen oberflächenaktiven Substanz gekennzeichnet sind.

Die oberflächenaktiven Substanzen im Sinne der Erfindung sind Homo-, Misch- oder Blockpolymerisate der Formel I

$$R_2Y - X_n - R_3 \qquad \text{I}$$

in der $X_n$ eine Kette von n Gliedern der Formeln II oder III

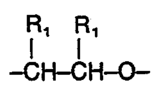

II

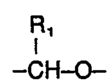

III

in beliebiger Reihenfolge und n 2–80, vorzugsweise 8–45 ist,

Y –O– oder –NH– ist,

$R_1$ H, $-CH_3$ oder $-C_2H_5$ ist, wobei die Reste $R_1$ gleich oder verschieden sein können, jedoch mindestens in der Hälfte der Kettenglieder X $-CH_3$ oder $-C_2H_5$ vorkommt, und in der $R_2$ und $R_3$ unabhängig voneinander H oder ein organischer Rest sind.

Besonders bevorzugt sind Verbindungen der Formel I, in denen $R_3$ Wasserstoff, Alkyl mit 1–20 C-Atomen, Carboxyalkyl mit 2–20 C-Atomen oder Alkylphenyl mit 1–10 Alkyl-C-Atomen und $R_2$ Wasserstoff oder Alkyl mit 1–20 C-Atomen, im Falle Y = –O– aber auch Carboxyalkyl mit 2–20 C-Atomen oder Alkylphenyl mit 1–10 Alkyl-C-Atomen bedeuten. Als insbesonders bevorzugt sind Verbindungen der Formel I, in welcher $R_3$ Wasserstoff oder Alkyl mit 1–20 C-Atomen und $R_2$ Wasserstoff bedeuten, hervorzuheben, wobei $R_2$ und $R_3$ = Wasserstoff die grösste Bedeutung zukommt.

Beispiele für die Reste $R_2$ und $R_3$ sind Wasserstoff, Methyl, Ethyl, Propyl, Butyl, oder die Reste, die sich vom Laurylalkohol oder Myristylalkohol ableiten; Carboxyalkylgruppen, die sich von der Essigsäure, Propionsäure, Buttersäure, Palmitinsäure oder Stearinsäure ableiten, Nonylphenyl oder Oleyl.

$R_2$ oder $R_3$ kann sich auch von einem mehrwertigen Alkohol wie Glycerin oder Pentaerythrit oder einer mehrwertigen Carbonsäure wie Citronensäure oder einem mehrwertigen Amin wie Äthylendiamin ableiten. Polyfunktionelle Glieder $R_2$ oder $R_3$ können mit drei oder mehreren Polyalkoxyketten der oben gezeigten Art verbunden sein, wobei bei tri- und höherfunktionellen Gliedern verzweigte Produkte entstehen.

Die Herstellung der erfindungsgemäss zu verwendenden oberflächenaktiven Substanzen werden in an sich bekannter Weise durch kontrollierte Addition von Alkylenoxiden an Alkylendiglykole (oder an mehrwertige Alkohole oder Amine, z.B. Pentaerythrit oder Äthylendiamin, zur Darstellung von verzweigten Produkten) hergestellt. Die endständigen Hydroxylfunktionen können gegebenenfalls anschliessend verestert oder veräthert werden. Eine allgemeine Vorschrift zur Herstellung eines geeigneten Blockpolymerisats ist in Beispiel 1a beschrieben.

Die erfindungsgemässen oberflächenaktiven Substanzen zeichnen sich dadurch aus, dass sie schon in Konzentrationen von 2–200 ppm in wässrigen Medien wirksam sind. Man kann sich vorstellen, dass diese Substanzen an der Grenzfläche so geformt sind, dass ihre hydrophoben Reste in die hydrophobe Phase ragen und die alternierend angeordneten hydrophilen Reste in die wässrige Phase. Da die Hydrophobizität der einzelnen hydrophoben Reste relativ schwach ist, dürfte auch die Bindung dieser einzelnen hydrophoben Reste an fremde hydrophobe Strukturen, z.B. an die hydrophoben Bereiche gelöster Proteine, so schwach sein, dass sie bei den erfindungsgemässen Anwendungskonzentrationen vernachlässigt werden können. Erst die Summe aller Bindungen der einzelnen hydrophoben Reste an eine grössere hydrophobe Fläche (Grenzfläche, gegen die die hydrophoben Bereiche der gelösten Proteine sehr klein sind) bewirkt, dass diese Substanzen auch bei geringer Anwendungskonzentration die Grenzflächen optimal bedecken.

Die Molekülform, die die erfindungsgemässen oberflächenaktiven Substanzen in wässriger Lösung zeigen, dürfte sich von der Form, die sie an der Oberfläche annehmen, unterscheiden. In wässriger Lösung ist die polymere Kette so geformt, dass die einzelnen hydrophoben Bereiche sich gegenseitig absättigen und die hydrophilen Bereiche nach aussen in die wässrige Umgebung ragen. Dies bewirkt eine ausreichend hohe Löslichkeit in wässrigen Medien, so dass ein genügend grosser Vorratspuffer vorhanden ist, der auch bei ständiger Änderung der Grösse der Grenzflächen eine optimale Beladung der Grenzflächen mit den erwähnten Substanzen ermöglicht.

Die Behinderung der Adsorption und Denaturierung durch die erfindungsgemässen Zusätze ist umso überraschender, als gerade, wie oben beschrieben, ein Zusatz von monomeren löslichen oberflächenaktiven Stoffen (Detergenzien), die Denaturierung von Proteinlösungen, insbesondere Insulin, beschleunigt.

Die erwähnten polymeren oberflächenaktiven Substanzen kann man entweder einer Proteinlösung zumischen oder man kann die Oberflächen, die mit Proteinlösungen in Berührung kommen sollen, mit diesen oberflächenaktiven Substanzen vorbehandeln.

Der Zusatz der erwähnten polymeren oberflächenaktiven Substanzen zu Proteinlösungen ist nicht auf Lösungen für therapeutische Zwecke begrenzt. Man kann diese Substanzen auch Proteinlösungen während des Herstellungs- und Reinigungsprozesses von Proteinen zusetzen zur Vermeidung von Adsorption und Denaturierung an Grenzflächen, insbesondere bei der Gelchromatographie und Ultrafiltration von Proteinlösungen.

Die Denaturierung von Insulin ist ein reversibler Prozess. Durch Behandeln des denaturierten Insulins mit leicht löslichen Detergenzien (z.B. Natriumdodecylsulfat), mit wässrigen alkalischen Medien oberhalb pH 10,5 oder mit konzentrierter Trifluoressigsäure konnten die denaturierten Produkte renaturiert werden. Es ist also möglich, die geringen Anteile von denaturiertem Insulin in den Insulinen, wie sie beim üblichen Herstellungsprozess anfallen, unter den genannten Bedingungen zu renaturieren, bevor man die Lösungen dieser Produkte mit den erfindungsgemässen oberflächenaktiven Substanzen in Berührung bringt.

Insulinlösungen für therapeutische Zwecke können nach folgender allgemeiner Herstellungsvorschrift hergestellt werden:

Bis zu 1 500 000 I.E. Rinder-, Schweine- oder Humaninsulin oder eines Des-B1-Phenylalanin-Derivates dieser Insuline, das bis zu 0,8 Gewichtsprozent Zink enthalten kann, werden in 400 ml Wasser unter Zugabe von 1n Salzsäure gelöst. Diese Lösung wird mit 500 ml einer Lösung gemischt, die ein Konservierungsmittel, z.B. Phenol, Kresol oder p-Hydroxybenzoesäuremethylester, ein Mittel zur Einstellung der Isotonie, z.B. Natriumchlorid, Glycerin, Glucose oder ein ähnliches Kohlenhydrat und ein Salz zur Pufferung des pH-Wertes, z.B. Natriumphosphat, Acetat, Citrat, 5,5-Diäthylbarbitalnatrium oder Tris-(hydroxymethyl)-aminomethan enthält. Daneben kann diese Lösung noch einen Depothilfsstoff, wie Surfen, zur Erzielung einer verzögerten Insulinwirkung enthalten. Mit 1n Salzsäure oder 1n Natronlauge wird ein pH-Wert von 3,0–4,0 oder 6,8–7,5 eingestellt. Anschliessend werden 50 ml einer wässrigen Lösung, die 2–200 mg einer der erfindungsgemässen oberflächenaktiven Substanzen enthält, zugefügt. Die Lösung wird mit Wasser auf 1,000 l ergänzt.

Eine solche Insulinlösung für therapeutische Zwecke kann mit einer Suspension, die amorphes oder kristallines Insulin mit verzögerter Wirkung enthält, gemischt werden.

Beispiel 1

a) In einem 10 l-Glaskolben mit Rührer, Heizbad, Rückflusskühler und einer Einrichtung zur Dosierung von Alkylenoxiden unter Stickstoff werden 152,1 g Propylenglykol und 125 g 40%ige Kalilauge vorgelegt. Durch Vakuumdestillation wird

entwässert. Anschliessend werden bei 120 °C langsam unter Rühren nacheinander 4141 g Propylenoxid und 476 g Äthylenoxid zugegeben. Nach beendeter Reaktion wird durch Zugabe von Milchsäure das Kaliumhydroxid neutralisiert. Durch Vakuumdestillation werden die leichtflüchtigen Anteile abgetrennt und das Produkt entwässert. Das mittlere Molekulargewicht des Produktes beträgt 2000 Dalton bei einem Gehalt an Polyoxyäthylen von 10 Gew.-% im Molekül.

b) 2 Proben mit jeweils 10 ml einer 0,1%igen Lösung von Rinderserum-Albumin bzw. Human-Albumin in 0,01 M Phosphatpuffer, pH 7, und 2 gleiche Proben mit Zusatz eines Stabilisators von 10 ppm (bezogen auf das Gewicht der Lösung) eines Blockpolymerisats, bestehend aus einer linearen Kette von Polypropylenglykol mit einem mittleren Molekulargewicht von 1750 Dalton, der beidseitig jeweils 5% Polyäthylenglykol anpolymerisiert waren, wurden bei 37 °C geschüttelt. Die beiden ersten Proben zeigten nach 7 Tagen bzw. 30 Tagen eine starke Trübung, hervorgerufen durch denaturiertes Protein. Die beiden Proben, die den Stabilisator enthielten, waren nach mehreren Monaten noch klar.

Beispiel 2

10 Proben mit jeweils 10 ml einer 0,01%igen Lösung des Enzyms β-Galaktosidase aus E. coli in 0,01 M Phosphatpuffer, pH 7, mit 3 × $10^6$ µU/ml wurden stufenweise mit 10–100 mg Silikonöl AK 350 versetzt. Die Suspensionen wurden 48 Stunden bei 5 °C geschüttelt. Dabei bildete sich eine Emulsion, die sich durch Ultrazentrifugation (40 000 g, 30 Minuten, 4 °C) in 2 Phasen trennen liess. In der wässrigen Phase wurde die Enzymaktivität bestimmt. Es zeigte sich, dass die Silikonölemulsion bis zu 70% des Enzyms adsorbiert hatte. Die Wiederholung des Versuchs in Gegenwart von 100 ppm (bezogen auf das Gewicht der Lösung) folgender Verbindung

$$CH_3-(CH_2)_{12}-CH_2-O-(-CH_2-CH_2-O-)_4-(-CH_2-\overset{\overset{CH_3}{|}}{C}H-O-)_4-H$$

ergab, dass in Gegenwart dieser oberflächenaktiven Verbindung keine Enzymaktivität an das Silikonöl adsorbiert wurde.

Beispiel 3

10 ml einer wässrigen Suspension von Polystyrol-Kugeln (Dow-Latex®) mit einem mittleren Durchmesser von 0,481 µm wurden in zwei gleiche Teile geteilt. Während der erste Teil unbehandelt blieb, wurde der zweite Teil mit 50 mg eines Blockpolymerisats, bestehend aus einer linearen Kette von Polypropylenglykol mit einem mittleren Molekulargewicht von 2250 Dalton, der beidseitig jeweils 5% Polyäthylenglykol anpolymerisiert waren, bei Raumtemperatur 2 Tage geschüttelt. Anschliessend wurde der Überschuss durch Dialyse gegen eine wässrige Lösung, die 20 ppm dieser polymeren oberflächenaktiven Substanz enthielt, entfernt.

Von den in der nachstehenden Tabelle angegebenen Proteinen wurden je 2 × 3 ml einer Lösung der genannten Konzentration in Phosphatpuffer pH 7 hergestellt. Jede dieser Lösungen wurde bei 5 °C mit jeweils 500 µl der unbehandelten und der wie oben beschrieben vorbehandelten Polystyrol-Suspension geschüttelt. Anschliessend wurde jede Probe durch ein 0,2 µm-Filter klarfiltriert. Die Tabelle gibt die Proteinkonzentrationen in den Filtraten an:

| Protein | Proteinkonzentration in den Lösungen A | B | C |
|---|---|---|---|
| Human-γ-Globulin | 1,2 mg/ml | 1,2 mg/ml | 0,2 mg/ml |
| Ei-Albumin | 0,5 mg/ml | 0,50 mg/ml | 0,22 mg/ml |
| Lysozym | 1,0 mg/ml | 0,95 mg/ml | 0,25 mg/ml |
| Secretin | 1,0 mg/ml | 1,0 mg/ml | 0,3 mg/ml |
| Glucagon | 1,0 mg/ml | 0,9 mg/ml | 0,3 mg/ml |
| Insulin | 1,0 mg/ml | 1,0 mg/ml | 0,2 mg/ml |

A = 3 ml der eingesetzten Proteinlösung + 450 µl Puffer
B = nach Kontakt mit vorbehandelten Polystyrolflächen
C = nach Kontakt mit unbehandelten Polystyrolflächen

Dieser Versuch wurde bei 37 °C wiederholt. Dabei wurden zusätzlich die Filtrate durch Gelchromatographie in einer Säule untersucht. Es zeigte sich, dass diejenigen Proteinlösungen, die mit den unbehandelten Polystyrolkugeln in Kontakt waren, hochmolekulare Aggregate enthielten. In den Proteinlösungen, die mit den vorbehandelten Polystyrolkugeln in Kontakt waren, wurden solche Aggregate nicht gefunden.

Beispiel 4

5 Proben mit jeweils 10 ml einer 0,1%igen Lösung von Glucagon in 0,05 M Tris/HCl-Puffer pH

8,0 und 5 gleiche Proben mit einem Zusatz von 50 ppm (bezogen auf das Gewicht der Lösung) folgender Verbindung:

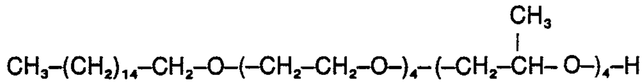

wurden bei Raumtemperatur geschüttelt. Die ersten 5 Proben zeigten nach 4 Tagen eine Trübung, verursacht durch ausgefallenes denaturiertes Protein. Diejenigen Proben, denen die oberflächenaktive Substanz zugesetzt worden war, blieben mehrere Wochen klar.

Beispiel 5

Ampullen mit 1–5 ml je einer 0,1%igen Lösung der Peptidhormone Secretin, Calcitonin, Glucagon, Gastrin, Adrenocorticotropes Hormon (ACTH), Bradykinin, Cholecystokinin (CCK), Gastrin Inhibierendes Polypeptid (GIP), Vasoaktives Intestinales Polypeptid (VIP) und Luteinisierungs-Releasing-Hormon (LHR) in 0,05 M Tris/HCl-Puffer pH 7,5 und gleiche Proben mit einem Zusatz von 10 ppm (bezogen auf das Gewicht der Lösung) eines Blockpolymerisats, bestehend aus einer linearen Kette von Polypropylenglykol mit einem mittleren Molekulargewicht von 2000 Dalton, der beidseitig jeweils 5% Polyäthylenglykol anpolymerisiert waren, wurden bei 37 °C geschüttelt. Die Proben ohne Stabilisator zeigten nach einigen Tagen eine starke Trübung, hervorgerufen durch denaturiertes Protein. Die stabilisierten Proben waren auch nach mehreren Monaten noch klar.

Beispiel 6

0,1%ige Lösungen von Ei-Albumin, Human-Immunglobulin-G bzw. Myoglobin vom Wal in 0,01 M Phosphatpuffer, pH 7, und gleiche Lösungen mit einem Zusatz von 0,1% (bezogen auf das Gewicht der Lösung) folgender Verbindung:

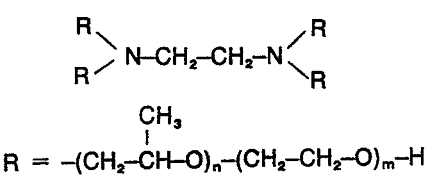

wobei n und m so gewählt wurden, dass ein mittleres Gesamtmolekulargewicht der obigen Verbindung von 12 500 Dalton resultierte und dass der Äthylenoxidanteil davon 40% betrug, wurden durch Ultrazentrifugation (z. B. 1000 g/90 Minuten) von Aggregaten befreit. Ampullen mit jeweils 10 ml dieser Lösungen wurden bei 37 °C geschüttelt. Die Proben ohne Stabilisator zeigten nach wenigen Stunden eine starke Trübung, verursacht durch ausgefallenes denaturiertes Protein. Diejenigen Proben, denen die oberflächenaktiven Substanzen zugesetzt worden waren, blieben mehrere Monate klar und zeigten in der analytischen Ultrazentrifuge keine neuen Aggregate.

Beispiel 7

Kristallisiertes Rinderinsulin (40 000 I. E.) mit 0,5 Gewichtsprozent Zink wurde in 200 ml Wasser unter Zugabe von 3 ml 1n Salzsäure gelöst. Diese Lösung wurde mit 700 ml einer Lösung von 1 g p-Hydroxybenzoesäuremethylester, 16 g Glycerin und 1,4 g Natriumacetat · 3 $H_2O$ versetzt. Die Lösung wurde mit 1n Natronlauge auf pH 6,9–7,4 eingestellt. Nach Zugabe von 5 ml einer wässrigen 0,1%igen Lösung von linearem Polypropylenglykol mit einem mittleren MG von 2000 Dalton wurde mit Wasser auf 1,000 l aufgefüllt und die Lösung sterilfiltriert.

Beispiel 8

Kristallisiertes Des-B1-Phenylalanininsulin vom Rind (100 000 I. E.) mit 0,6 Gewichtsprozent Zink wurde in 200 ml Wasser unter Zugabe von 3 ml 1 n Salzsäure gelöst. Diese Lösung wurde mit 700 ml einer Lösung von 2 g Phenol, 17 g Glycerin und 6,057 g Tris-(hydroxymethyl)-aminomethan, die mit 35 ml 1n Salzsäure auf pH 7,6 eingestellt worden war, versetzt. Die Lösung wurde auf pH 7,2–7,6 eingestellt. Nach Zugabe von 10 ml einer wässrigen 1%igen Lösung der Verbindung

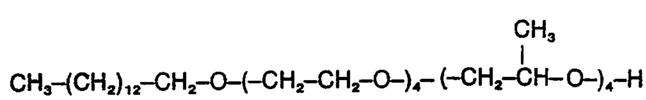

wurde mit Wasser auf 1,000 l aufgefüllt und die Lösung sterilfiltriert.

Beispiel 9

Amorphes Rinderinsulin (1 000 000 I. E.) mit 0,8 Gewichtsprozent Zink wurde in 400 ml Wasser unter Zugabe von 5 ml 1n Salzsäure gelöst. Diese Lösung wurde mit 500 ml einer Lösung von 2,5 g Phenol, 16 g Glycerin und 1,78 g $Na_2HPO_4$ · 2 $H_2O$ versetzt. Die Lösung wurde auf pH 7,2–7,5 eingestellt. Nach Zugabe von 5 ml einer wässrigen 0,1%igen Lösung von linearem Polyproylenglykol mit einem mittleren MG von 1750 Dalton wurde mit Wasser auf 1,000 l aufgefüllt und die Lösung sterilfiltriert.

Beispiel 10

Rinderinsulin (40 000 I. E.), das in Gegenwart von einem Blockpolymerisat, bestehend aus einer linearen Kette von Polypropylenglykol mit einem mittleren MG von 1750 Dalton, der beidseitig jeweils 5% Polyäthylenglykol anpolymerisiert waren, durch Chromatographie gereinigt worden war und 0,6 Gewichtsprozente Zink enthielt, wurde in 200 ml Wasser unter Zugabe von 3 ml 1n Salzsäure gelöst. Diese Lösung wurde mit 700 ml

einer Lösung von 2,5 g m-Kresol, 50 g Glucose, 1,4 g Natriumacetat · 3 $H_2O$ und 10 mg des oben genannten Blockpolymerisats versetzt. Die Lösung wurde mit 1n Natronlauge auf pH 6,9–7,4 eingestellt, mit Wasser auf 1,000 l ergänzt und sterilfiltriert.

Beispiel 11

Kristallisiertes Schweineinsulin (40 000 I.E.) mit 0,6 Gewichtsprozent Zink wurde in 200 ml Wasser unter Zugabe von 3 ml 1n HCl gelöst. Diese Lösung wurde mit 700 ml einer Lösung von 1 g p-Hydroxybenzoesäuremethylester, 17 g Glycerin, 1,4 g Natriumacetat · 3 $H_2O$ und 10 mg linearem Polypropylenglykol mit einem mittleren MG von 1750 Dalton versetzt. Die Lösung wurde auf pH 6,9–7,4 eingestellt. Mit Wasser wurde auf 1,000 l ergänzt und die Lösung sterilfiltriert.

Beispiel 12

Kristallisiertes Rinderinsulin (450 000 I.E.), das in Gegenwart eines Blockpolymerisats, bestehend aus einer linearen Kette von Polypropylenglykol mit einem mittleren MG von 1750 Dalton, der beidseitig jeweils 5% Polyäthylenglykol anpolymerisiert waren, durch eines der üblichen Chromatographie-Verfahren gereinigt worden war und 0,5 Gewichtsprozent Zink enthielt, wurde in 400 ml 0,03 n Salzsäure gelöst und diese Lösung mit einer Lösung von 150 mg $ZnCl_2$ und 5 mg des für die Chromatographie verwendeten Blockpolymerisats in 100 ml 0,03 n HCl versetzt. Die Lösung wurde sterilfiltriert und mit 500 ml einer ebenfalls sterilfiltrierten Lösung von 70 g NaCl, 14 g Natriumacetat · 3 $H_2O$, 5 mg des für die Chromatographie verwendeten Blockpolymerisats und 10 ml 1n NaOH in Wasser gemischt. Die Mischung wurde auf pH 5,4 eingestellt und 48 Stunden bei Raumtemperatur gerührt, wobei das Insulin in Rhomboedern kristallisierte. Die Kristallsuspension wurde mit 10,25 l einer sterilen Lösung von 20 g NaCl, 1,75 g Natriumacetat · 3 $H_2O$, 11,25 g p-Hydroxybenzoesäuremethylester und 102,5 mg des für die Chromatographie verwendeten Blockpolymerisats versetzt. Durch Zutropfen von 1n NaOH wurde pH 6,9–7,3 eingestellt.

Beispiel 13

Kristallisiertes Rinderinsulin (40 000 I.E.), das in Gegenwart der Verbindung

$$CH_3\cdot(CH_2)_{10}-CH_2-O-(-CH_2-CH_2-O-)_4-(-CH_2-\overset{\displaystyle CH_3}{\overset{|}{CH}}-O-)_4-H$$

durch eines der üblichen Chromatographieverfahren gereinigt worden war und 0,5 Gewichtsprozent Zink enthielt, wurde in 200 ml Wasser unter Zugabe von 3 ml 1n Salzsäure gelöst. Diese Lösung wurde mit 700 ml einer Lösung von 1 g p-Hydroxybenzoesäuremethylester, 50 g Glucose, 0,175 g Surfen® und 100 mg der gleichen Substanz, wie für die Chromatographie verwendet, versetzt. Die Lösung wurde gegebenenfalls mit 1n HCl oder 1n NaOH auf pH 3,3–3,5 eingestellt, mit Wasser auf 1,000 l ergänzt und sterilfiltriert.

**Patentansprüche**

für die Vertragsstaaten BE CH DE FR GB IT LI NL SE

1. Wässrige Proteinlösung, gekennzeichnet durch einen Gehalt an einem oberflächenaktiven Homopolymerisat, Mischpolymerisat oder Blockpolymerisat der Formel I,

$$R_2Y-X_n-R_3 \qquad I$$

in der $X_n$ eine Kette von n Gliedern der Formeln II oder III

$$\underset{\displaystyle II}{-\overset{\displaystyle R_1}{\overset{|}{CH}}-\overset{\displaystyle R_1}{\overset{|}{CH}}-O-} \qquad\qquad \underset{\displaystyle III}{-\overset{\displaystyle R_1}{\overset{|}{CH}}-O-}$$

in beliebiger Reihenfolge und n 2–80, vorzugsweise 8–45 ist,

Y –O– oder –NH– ist,

$R_1$ H, $-CH_3$ oder $-C_2H_5$ ist, wobei die Reste $R_1$ gleich oder verschieden sein können, jedoch mindestens in der Hälfte der Kettenglieder X $-CH_3$ oder $-C_2H_5$ vorkommt, und in der

$R_2$ und $R_3$ unabhängig voneinander H oder ein organischer Rest sind.

2. Wässrige Proteinlösung gemäss Anspruch 1, dadurch gekennzeichnet, dass $R_2$ und $R_3$ je Alkyl mit 1–20 C-Atomen, Carboxalkyl mit 2–20 C-Atomen oder Alkylphenyl mit 1–10 Alkyl-C-Atomen bedeuten, $R_2$ jedoch, falls Y –NH– bedeutet, nur Alkyl mit 1–20 C-Atomen sein kann.

3. Wässrige Proteinlösung gemäss Anspruch 1, dadurch gekennzeichnet, dass $R_2$ und/oder $R_3$ drei- oder mehrwertig sind und mit drei oder mehreren Polyalkoxyketten $-X_n$ zu verzweigten Produkten verbunden sind.

4. Wässrige Proteinlösung gemäss Anspruch 1, dadurch gekennzeichnet, dass das Protein Insulin ist und dass die Lösung gegebenenfalls die bei Insulinlösungen üblichen Zusätze zur Einstellung der Isotonie, Konservierung und/oder Depot-Wirkung enthält.

5. Verfahren zur Herstellung einer stabilen Proteinlösung, dadurch gekennzeichnet, dass man einer wässrigen Proteinlösung eine oberflächenaktive Substanz gemäss Ansprüchen 1–3 zusetzt.

6. Verfahren zur Herstellung einer Insulinlösung, dadurch gekennzeichnet, dass man eine Lösung aus Rinder-, Schweine- oder Humaninsulin oder einem Des-B1-Phenylalaninderivat dieser Insuline, die bis zu 0,8% Zink, bezogen auf

das Insulingewicht, enthält, mit einer Lösung, die eine Verbindung der Formel I enthält, mischt und gegebenenfalls ein Mittel zur Einstellung der Isotonie wie Glycerin, Natriumchlorid, Glucose oder ein ähnliches Kohlenhydrat, zur Konservierung einen Zusatz von Phenol, Kresol oder p-Hydroxybenzoesäuremethylester, zur Pufferung des pH-Wertes Natriumphosphat, Acetat, Citrat, 5,5-Diäthylbarbitalnatrium oder Tris-(hydroxymethyl)-aminomethan zusetzt.

7. Verfahren zur Herstellung eines Insulinpräparates mit Depotwirkung, dadurch gekennzeichnet, dass man eine Insulinlösung, hergestellt gemäss Anspruch 6, mit einer verzögert wirkenden Depotkomponente des Insulins versetzt.

8. Verfahren zur Behandlung von hydrophoben Oberflächen zur Beseitigung ihrer absorbierenden und denaturierenden Wirkung auf Proteine, dadurch gekennzeichnet, dass man die Oberflächen mit einer wässrigen Lösung einer oberflächenaktiven Substanz der in Anspruch 1 angegebenen allgemeinen Formel I behandelt.

9. Verwendung von Lösungen nach Ansprüchen 1–3 bei der Reinigung von Proteinen durch Chromatographie oder Ultrafiltration.

10. Verwendung von Lösungen nach Anspruch 4 zum Zwecke der Reinigung von Insulin durch Chromatographie oder Kristallisation.

11. Verwendung von oberflächenaktiven Substanzen der in Anspruch 1 angegebenen allgemeinen Formel I zur Vorbehandlung von hydrophoben Oberflächen zur Vermeidung der Adsorption oder Denaturierung von Proteinen, insbesondere Insulin.

**Patentansprüche:**
für den Vertragsstaat AT

1. Verfahren zur Herstellung einer stabilen Proteinlösung, dadurch gekennzeichnet, dass man einer wässrigen Proteinlösung ein oberflächenaktives Homopolymerisat, Mischpolymerisat oder Blockpolymerisat der Formel I zusetzt,

$$R_2Y - X_n - R_3 \qquad I$$

in der $X_n$ eine Kette von n Gliedern der Formeln II oder III

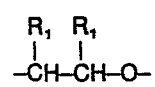

II

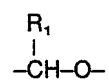

III

in beliebiger Reihenfolge und n 2–80, vorzugsweise 8–45 ist,

Y –O– oder –NH– ist,

$R_1$ H, $-CH_3$ oder $-C_2H_5$ ist, wobei die Reste $R_1$ gleich oder verschieden sein können, jedoch mindestens in der Hälfte der Kettenglieder X $-CH_3$ oder $-C_2H_5$ vorkommt, und in der

$R_2$ und $R_3$ unabhängig voneinander H oder ein organischer Rest sind.

2. Verfahren gemäss Anspruch 1, dadurch gekennzeichnet, dass $R_2$ und $R_3$ je Alkyl mit 1–20 C-Atomen, Carboxalkyl mit 2–20 C-Atomen oder Alkylphenyl mit 1–10 Alkyl-C-Atomen bedeuten, $R_2$ jedoch, falls Y –NH– bedeutet, nur Alkyl mit 1–20 C-Atomen sein kann.

3. Verfahren gemäss Anspruch 1, dadurch gekennzeichnet, dass $R_2$ und/oder $R_3$ drei oder mehrwertig sind und mit drei oder mehreren Polyalkoxyketten $-X_n$ zu verzweigten Produkten verbunden sind.

4. Verfahren gemäss Anspruch 1, dadurch gekennzeichnet, dass das Protein Insulin ist und dass die Lösung gegebenenfalls die bei Insulinlösungen üblichen Zusätzen zur Einstellung der Isotonie, Konservierung und/oder Depot-Wirkung, enthält.

5. Verfahren nach Anspruch 4, dadurch gekennzeichnet, dass man eine Lösung aus Rinder-, Schweine- oder Humaninsulin oder einem Des-B1-Phenyl-alaninderivat dieser Insuline, die bis zu 0,8% Zink, bezogen auf das Insulingewicht, enthält, mit einer Lösung, die eine Verbindung der Formel I enthält, mischt und gegebenenfalls ein Mittel zur Einstellung der Isotonie wie Glycerin, Natriumchlorid, Glucose oder ein ähnliches Kohlenhydrat, zur Konservierung einen Zusatz von Phenol, Kresol oder p-Phydroxybenzoesäuremethylester, zur Pufferung des pH-Wertes Natriumphosphat, Acetat, Citrat, 5,5-Diäthylbarbitalnatrium oder Tris-(hydroxymethyl)-aminomethan zusetzt.

6. Verfahren zur Herstellung eines Insulinpräparates mit Depotwirkung, dadurch gekennzeichnet, dass man eine Insulinlösung, hergestellt gemäss Anspruch 5, mit einer verzögert wirkenden Depotkomponente des Insulins versetzt.

7. Verfahren zur Behandlung von hydrophoben Oberflächen zur Beseitigung ihrer absorbierenden und denaturierenden Wirkung auf Proteine, dadurch gekennzeichnet, dass man die Oberflächen mit einer wässrigen Lösung einer oberflächenaktiven Substanz der Formel I, in der $R_2$, $R_3$, X, Y und n die in Anspruch 1 definierte Bedeutung haben, behandelt.

8. Verwendung von nach Ansprüchen 1–3 hergestellten Lösungen bei der Reinigung von Proteinen durch Chromatographie oder Ultrafiltration.

9. Verwendung von nach Anspruch 4 hergestellten Lösungen zum Zwecke der Reinigung von Insulin durch Chromatographie oder Kristallisation.

10. Verwendung von oberflächenaktiven Substanzen der Formel I, in der $R_2$, $R_3$, X, Y und n die in Anspruch 1 definierte Bedeutung haben, zur Vorbehandlung von hydrophoben Oberflächen zur Vermeidung der Adsorption oder Denaturierung von Proteinen, insbesondere Insulin.

**Claims**
for contracting-state Austria

1. Process for the preparation of a stable protein solution, which comprises adding to an aqueous

protein solution a surface-active homo-, co- or block-polymerizate of the formula I

$$R^2Y - X_n - R^3 \qquad I$$

in which $X_n$ denotes a chain of n members of the formulae II and III

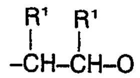

II

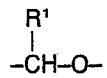

III

in any desired sequence and n is 2 to 80, preferably 8 to 45,

Y is –O– or –NH–

$R^1$ is H, $-CH_3$ or $-C_2H_5$, the radicals $R^1$ being identical or different, but in at least one half of the chain members X contains $-CH_2$ or $-C_2H_5$, and

$R^2$ and $R^3$, independently of each other, are hydrogen or an organic radical.

2. Process as claimed in claim 2, characterized in that $R^2$ and $R^3$ each denote alkyl with 1 to 20 carbon atoms, carboxyalkyl with 2 to 20 carbon atoms or alkylphenyl with 1 to 10 alkyl carbon atoms, with the proviso that $R^2$ can only be alkyl with 1 to 20 carbon atoms in the case of Y being –NH–.

3. Process as claimed in claim 1, wherein $R^2$ and/or $R^3$ are three- or polyvalent and linked with three or more polyalkoxy chains $-X_n$ to give branched products.

4. Process as claimed in claim 1, characterized in that the protein is insulin and the solution optionally contains the usual additives for insulin solutions to adjust isotonicity, for preservation and/or for achieving a depot effect.

5. Process as claimed in claim 4, characterized in that a solution of bovine insulin, swine insulin or human insulin, or of a De-B1-phenylalanine derivative of these insulins, which contains up to 0.8% of zinc, based on the weight of insulin, is mixed with a solution which contains a compound of the formula I and, optionally, an agent to render the solution isotonic, such as glycerol, sodium chloride, glucose or a similar carbohydrate, a preservative, such as phenol, cresol or methyl p-hydroxybenzoate, and/or an agent for buffering the pH value, such as sodium phosphate, acetate, citrate, sodium 5,5-diethylbarbital or tris-(hydroxymethyl)-aminomethane.

6. Process for the preparation of an insulin formulation with a depot action characterized in that a depot component of the insulin, which provides a delayed action is added to an insulin solution prepared according to claim 5.

7. Process for the treatment of a hydrophobic surface to abolish its adsorbing and denaturing effect on proteins, which comprises treating the said surface with an aqueous solution of a surface-active substance of the formula I, wherein $R_2$, $R_3$, X, Y and n are as defined in claim 1.

8. Use of solutions prepared according to claims 1 to 3 for the purpose of purifying a protein by chromatography or crystallization.

9. Use of solutions prepared according to claim 4 for the purpose of purifying insulin by chromatography or crystallization.

10. Use of surface active substances of the formula I wherein $R_2$, $R_3$, X, Y and n are as defined in claim 1 for the pre-treatment of a hydrophobic surface in order to prevent the adsorption or denaturation of proteins, in particular insulin thereon.

**Claims:**

for the contracting states: BE, CH, LI, DE, FR, GB, IT, NL, SE

1. Aqueous protein solution characterized by a content of a surface-active homo-, co- or block-polymerizate of the formula I

$$R^2Y - X_n - R^3$$

in which $X_n$ denotes a chain of n members of the formulae II and III

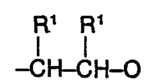

II

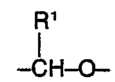

III

in any desired sequence and n is 2 to 80, preferably 8 to 45,

Y is –O– or –NH–

$R^1$ is H, $-CH_3$ or $-C_2H_5$, the radicals $R^1$ being identical or different, but in at least one half of the chain members X contains $-CH_2$ or $-C_2H_5$, and

$R^2$ and $R^3$, independently of each other, are hydrogen or an organic radical.

2. Aqueous protein solution as claimed in claim 1, characterized in that $R^2$ and $R^3$ each denote alkyl with 1 to 20 carbon atoms, carboxyalkyl with 2 to 20 carbon atoms or alkylphenyl with 1 to 10 carbon atoms, with the proviso that $R^2$ can only be alkyl with 1 to 20 carbon atoms in the case of Y being –NH–.

3. Aqueous protein solution as claimed in claim 1, characterized in that $R^2$ and/or $R^3$ are three- or polyvalent and linked with three or more polyalkoxy chains $-X_n$ to give branched products.

4. Aqueous protein solution as claimed in claim 1, characterized in that the protein is insulin and the solution optionally contains the usual additives for insulin solutions to adjust isotonicity, for preservation and/or for achieving a depot effect.

5. Process for the preparation of a stable protein solution, which comprises adding to an aqueous protein solution a surface-active substance as claimed in claims 1 to 3.

6. Process for the preparation of an insulin solution, characterized in that a solution of bovine insulin, swine insulin or human insulin, or of a De-B1-phenylalanine derivative of these insulins, which contains up to 0.8% of zinc, based on the

weight of insulin, is mixed with a solution which contains a compound of the formula I and, optionally, an agent to render the solution isotonic, such as glycerol, sodium chloride, glucose or a similar carbohydrate, a preservative, such as phenol, cresol or methyl p-hydroxybenzoate, and/or an agent for buffering the pH value, such as sodium phosphate, acetate, citrate, sodium 5,5-diethylbarbital or tris-(hydroxymethyl)-aminomethane.

7. Process for the preparation of an insulin formulation with a depot action, characterized in that a depot component of the insulin, which provides a delayed action is added to an insulin solution prepared according to claim 6.

8. Process for the treatment of a hydrophobic surface to abolish its adsorbing and denaturing effect on proteins, which comprises treating the said surface with an aqueous solution of a surface-active substance of the formula I as defined in claim 1.

9. Use of a solution according to claims 1 to 3 for the purpose of purifying a protein by chromatography or crystallization.

10. Use of a solution according to claim 4 for the purpose of purifying insulin by chromatography or crystallization.

11. Use of a surface-active substance of the formula I as defined in claim 1 for the pretreatment of a hydrophobic surface in order to prevent the adsorption or denaturation of proteins, in particular insulin thereon.

**Revendications**

pour les Etats contractants: BE, CH, LI, DE, FR, GB, IT, NL, SE

1. Solution aqueuse de protéine, caractérisée en ce qu'elle contient un homopolymérisat, un copolymérisat ou un polymérisat séquencé tensioactif, de formule I

$$R_2Y - X_n - R_3 \qquad I$$

dans laquelle $X_n$ représente une chaîne de n éléments de formule II ou III

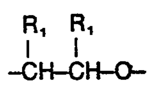

II

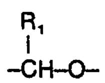

III

dans un ordre quelconque, et n est compris entre 2 et 80, de préférence entre 8 et 45,

Y est –O– ou –NH–,

$R_1$ est H, $-CH_3$ ou $C_2H_5$, auquel cas les radicaux $R_1$ peuvent être identiques ou différents, mais au moins dans la moitié des éléments de la chaîne X représente $-CH_3$ ou $-C_2H_5$ et dans laquelle

$R_2$ et $R_3$ sont indépendamment l'un de l'autre l'hydrogène ou un radical organique.

2. Solution aqueuse de protéine selon la revendication 1, caractérisée en ce que $R_2$ et $R_3$ représentent chacun un groupe alcoyle ayant de 1 à 20 atomes de carbone, carboxyalcoyle ayant de 2 à 20 atomes de carbone ou alcoylphényle ayant de 1 à 10 atomes de carbone dans la partie alcoyle, mais si Y est –NH–, $R_2$ ne peut être qu'un groupe alcoyle ayant de 1 à 20 atomes de carbone.

3. Solution aqueuse de protéine selon la revendication 1, caractérisée en ce que $R_2$ et/ou $R_3$ sont trivalents ou plus et sont reliés par au moins 3 chaînes polyalcoxy $-X_n$ en des produits ramifiés.

4. Solution aqueuse de protéine selon la revendication 1, caractérisée en ce que la protéine est l'insuline et la solution contient éventuellement les additifs usuels pour les solutions d'insuline pour régler l'isotonie, la conservation et/ou l'effet retard.

5. Procédé pour la préparation d'une solution stable de protéine, caractérisé en ce qu'à une solution aqueuse de protéine on ajoute une substance tensioactive selon l'une quelconque des revendications 1 à 3.

6. Procédé pour la préparation d'une solution d'insuline, caractérisé en ce qu'on mélange une solution d'insuline de bœuf, de porc ou humaine ou un dérivé des-B1-phénylalanine de ces insulines, qui contient jusqu'à 0,8% de zinc par rapport au poids d'insuline, avec une solution qui contient un composé de formule I et éventuellement on ajoute un agent pour le réglage de l'isotonie tel que la glycérine, le chlorure de sodium, le glucose ou un hydrate de carbone similaire, pour la conservation: le phénol, le crésol ou l'ester méthylique de l'acide p-hydroxybenzoïque, pour le tamponnage de la valeur du pH, le phosphate, l'acétate, le citrate de sodium, le 5,5-diéthylbarbital sodique ou le tris-(hydroxyméthyl)-aminométhane.

7. Procédé pour la préparation d'une préparation d'insuline ayant un effet retard, caractérisé en ce qu'à une solution d'insuline préparée selon la revendication 6, on ajoute un constituant retard de l'insuline ayant un effet retard.

8. Procédé pour le traitement de surfaces hydrophobes pour éliminer leur action adsorbante et dénaturante sur les protéines, caractérisé en ce qu'on traite les surfaces avec une solution aqueuse d'une substance tensioactive de formule générale I indiquée dans la revendication 1.

9. Utilisation des solutions selon l'une quelconque des revendications 1 à 3 pour la purification des protéines par chromatographie ou ultrafiltration.

10. Utilisation des solutions selon la revendication 4 pour la purification de l'insuline par chromatographie ou cristallisation.

11. Utilisation des substances tensioactives de formule générale I donnée dans la revendication 1, pour le prétraitement des surfaces hydrophobes de manière à éviter l'adsorption ou la dénaturation des protéines, en particulier de l'insuline.

**Revendication**

pour l'Etat contractant Autriche

1. Procédé pour la préparation d'une solution stable de protéine, caractérisé en ce qu'à une solution aqueuse de protéine, on ajoute un homopolymérisat, un copolymérisat, ou un polymérisat séquencé, tensioactif, de formule I.

$$R_2y - X_n - R_3 \qquad I$$

dans laquelle $X_n$ représente une chaîne de n éléments de formule II ou III

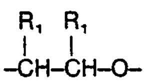

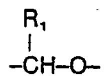

II III

dans un ordre quelconque et n est compris entre 2 et 80, de préférence entre 8 et 45,

Y est –O– ou –NH–,

$R^1$ est H, $-CH_3$, ou $-C_2H_5$ auquel cas les radicaux $R_1$ peuvent être identiques ou différents, mais au moins dans la moitié des éléments de la chaîne X représente $-CH_3$ ou $-C_2H_5$, et dans laquelle

$R_2$ et $R_3$ sont indépendamment l'un de l'autre l'hydrogène ou un radical organique.

2. Procédé selon la revendication 1, caractérisé en ce que $R_2$ et $R_3$ représentent chacun un groupe alcoyle ayant de 1 à 20 atomes de carbone, carboxyalcoyle ayant de 2 à 20 atomes de carbone ou alcoylphényle ayant de 1 à 10 atomes de carbone dans la partie alcoyle, mais si Y représente –NH–, $R_2$ ne peut être qu'un groupe alcoyle ayant de 1 à 20 atomes de carbone.

3. Procédé selon la revendication 1, caractérisé en ce que $R_2$ et/ou $R_3$ sont trivalents ou plus et sont reliés par au moins 3 chaînes polyalcoxy $-X_n$ en des produits ramifiés.

4. Procédé selon la revendication 1, caractérisé en ce que la protéine est l'insuline et la solution contient éventuellement les additifs usuels pour les solutions d'insuline, pour le réglage de l'isotonie, la conservation et/ou l'effet retard.

5. Procédé selon la revendication 4, caractérisé en ce qu'on mélange une solution d'insuline de bœuf, de porc ou humaine ou un dérivé des-BI-phénylalanine de ces insulines, qui contient jusqu'à 0,8% de zinc par rapport au poids d'insuline, avec une solution qui contient un composé de formule I et éventuellement on ajoute un agent pour le réglage de l'isotonie tel que la glycérine, le chlorure de sodium, le glucose ou un hydrate de carbone similaire, pour la conservation: le phénol, le crésol ou l'ester méthylique de l'acide p-hydroxybenzoïque, pour le tamponnage de la valeur du pH: le phosphate, l'acétate, le citrate de sodium, le 5,5-diéthylbarbital sodique ou le tris-(hydroxyméthyl)-aminométhane.

6. Procédé pour la préparation d'une préparation d'insuline avec un effet retard, caractérisé en ce qu'à une solution d'insuline préparée selon la revendication 5, on ajoute un constituant retard de l'insuline ayant un effet retard.

7. Procédé pour le traitement des surfaces hydrophobes pour éliminer leur action adsorbante et dénaturante sur les protéines, caractérisé en ce qu'on traite les surfaces avec une solution aqueuse d'une substance tensioactive de formule I dans laquelle $R_2$, $R_3$, X, Y et n ont les significations définies dans la revendication 1.

8. Utilisation de solutions préparées selon l'une quelconque des revendications 1 à 3, pour la purification des protéines par chromatographie ou ultrafiltration.

9. Utilisation des solutions préparées selon la revendication 4 pour la purification de l'insuline par chromatographie ou cristallisation.

10. Utilisation des substances tensioactives de formule I, dans lesquelles $R_2$, $R_3$, X, Y et n ont les significations définies dans la revendication 1, pour le prétraitement des surfaces hydrophobes pour éviter l'adsorption ou la dénaturation des protéines, en particulier de l'insuline.